# EUROPÄISCHE PATENTSCHRIFT

(11) **EP 1 286 675 B1**
(45) Veröffentlichungstag und Bekanntmachung des Hinweises auf die Patenterteilung: **12.10.2005**
(21) Anmeldenummer: 01937841.3
(22) Anmeldetag: 23.05.2001
(51) Int. Cl.: A61K 33/04

(54) **VERWENDUNG VON SELENITHÄLTIGEN WÄSSERIGEN LÖSUNGEN ZUR HERSTELLUNG EINES MEDIKAMENTS ZUR BEHANDLUNG VIRALER ERKRANKUNGEN UND PIGMENTFLECKEN**
USE OF AQUEOUS SELENITE SOLUTIONS FOR THE MANUFACTURE OF A MEDICAMENT FOR THE TREATMENT OF VIRAL DISEASES AND PIGMENTED SPOTS
UTILISATION DE SOLUTIONS AQUEUSES CONTENANT DU SELENITE POUR LE TRAITEMENT DES MALADIES VIRALES ET POUR LE TRAITEMENT DES TACHES PIGMENTAIRES

(30) Priorität: 05.06.2000 AT 9782000
(43) Veröffentlichungstag der Anmeldung: 05.03.2003
(73) Patentinhaber: Vis-Vitalis Lizenz- und Handels AG, 5081 Anif (AT)
(72) Erfinder: KÖSSLER, Peter, A-5571 Mariapfarr (AT); FUCHS, Norbert, A-5571 Mariapfarr (AT); KUKLINSKI, Bodo, D-18055 Rostock (DE); SCHIEFER, Raimund, A-5580 Tamsweg (AT)
(74) Vertreter: Alge, Daniel
(86) Internationale Anmeldenummer: PCT/AT2001/000162
(87) Internationale Veröffentlichungsnummer: WO 2001/093910

(56) Entgegenhaltungen:
- EP-A- 0 913 155
- DE-C- 4 413 839
- GB-A- 2 323 030
- US-A- 4 668 515

## Beschreibung

Die Erfindung basiert auf der Erhöhung des antioxidativen Potentials selenhältiger wässriger Lösungen sowie pharmazeutisch verabreichbare oder lebensmittelkompatible Selenpräparate.

Sämtliche Stoffwechselprozesse in organischen Lebewesen (Pflanze, Tier, Mensch) im Sinne von Wachstums-; Differenzierungs- und Energie-Prozessen sind auf biochemischer Ebene ein Wechselspiel von reduktiven und oxidativen Prozessen. Diese "Redox-Vorgänge" sind letztlich Ausdruck der Elektronen-Übertragung von biochemischen Reduktions-Äquivalenten, wie z.B. NADH + H⁺ (Elektronendonator) auf atmosphärischen, molekularen Sauerstoff als Oxidationsmittel (Elektronen-Akzeptor). Die Oxidation unserer Nährstoffe (Fette, Kohlenhydrate, Proteine, Sauerstoff) dient der permanenten Erhaltung und Entwicklung unserer biologischen Strukturen.

Andererseits aber bestehen gerade unsere zellulären und subzellulären Strukturen, die daraus gebildeten Gewebe und Organe und letztlich jedes organische Individuum in ihrer Gesamtheit aus gerade jenen Strukturen (Nährstoffen), die zur Erhaltung und Entwicklung lebender Organismen ständig von außen zugeführt, zu Energiegewinnung oxidiert, sogleich aber auch zur Erhaltung der funktionellen, anatomischen und histologischen Struktur dienen müssen. Somit sind diese biologischen Strukturen letztlich ebenso oxidabel wie jene Nährstoffe, die zur Aufrechterhaltung unserer Lebensenergie oxidiert werden müssen. Um eine "Autooxidation" biologischer Strukturen zu verhindern, bedient sich der organische lebende Organismus endogener und exogener "Antioxidantien". Zu den endogenen Antioxidantien gehören unter anderem Enzyme und Enzym-Systeme, wie die Superoxiddismutase, Katalasen, Peroxydasen, Cholesterin und reduziertes Glutathion, während exogene Antioxidantien, z.B. Vitamin A, β-Carotin, Vitamin E, Vitamin C oder Selen darstellen.

Das Maß für die "antioxidative Kapazität", also die Bereitschaft, Elektronen auf andere Atome und Moleküle zu übertragen, wird im sogenannten "Reduktionspotential" (Standard-Redoxpotential) quantitativ ausgedrückt. Nachfolgende Tabelle gibt einen Überblick über die Standard-Redoxpotentiale einiger endogener und exogener Antioxidantien organischen Lebewesen:

| **Standard-Redoxpotentiale einiger Nährstoffe** | |
|---|---|
| **E**_{**o**} **(Volt)** | **System** |
| + 0,82 | O₂/H₂O |
| + 0,366 (basisches Milieu) | Selenit |
| + 0,300 | Tocopherol (Vitamin E) |
| + 0,100 | Ubiquinon (Coenzym Q₁₀) |
| + 0,08 | Ascorbinsäure |
| + 0 (+0,16 bis -0,02 V) | Flavonoide |
| - 0,12 | Riboflavin (Vitamin B₂) |
| - 0,22 | Cystin/Cystein |
| - 0,23 | G SH/GSSG |
| - 0,29 | Thioctsäure (α-Liponsäure) |
| - 0,32 | NADH + H⁺/NAD |
| - 0,740 (saures Milieu) | Selenit |

Antioxidantien sind somit Atome und Moleküle (für den humanen Organismus, vor allem Nährstoffmoleküle und Enzym-Komplexe), die schneller mit Stöffwechselradikalen reagieren als biologische Strukturen. Sie schützen somit unsere Zell-, Gen- und Bindegewebs-Strukturen dadurch, dass sie Stoffwechselzündfunken (Radikale, Peroxide) abfangen, bevor diese z.B. ungesättigte Fettsäuren unserer Biomembranen oder schwefelhaltige Bauteile lebenswichtiger Struktur- oder Enzymproteine angreifen. Wie oben stehende Tabelle zeigt, verändern bestimmte Elemente, wie z.B. Selen, ihr Standard-Redopotential durch Veränderung des pH-Milieus, indem diese Verbindungen gelöst sind.

Selen ist ein essentielles Spurenelement für höhere Tiere und den Menschen. Es besitzt eine Schutzfunktion für Proteine gegen Oxidation, die z.B. durch die Glutathion-Peroxidase erfolgt, die die Aminosäure Selenocystein im aktiven Zentrum enthält. Selenmangel wird mit Rheumatismus und Grauem Star in Verbindung gebracht, die Keshan-Krankheit, die in einigen Gebieten Chinas verbreitet ist, gilt als Selen-Mangelerscheinung. Selenite können die Wirkung von Vitamin E steigern und sorgen für die Entgiftung von Quecksilber und Cadmium. Eine Schutzwirkung von Selen vor Karzinogenen wird ebenfalls postuliert.

Andererseits wirkt Selen in höheren Konzentrationen toxisch, wobei die Toxizität darauf zurückgeführt wird, dass Selen den Schwefel in Proteinen verdrängen kann. Die Ausscheidung erfolgt in der Regel als Selenat über die Niere und den Darm. Erkrankungen des menschlichen Körpers werden hervorgerufen, wenn die tägliche Nahrung mehr als 1 µg Selen/g enthält (wobei ein Mindestge halt von 0,02 µg Selen/g erforderlich ist, um Mangelerscheinungen vorzubeugen). Insgesamt enthält der menschliche Körper ca. 10 bis 15 µg Selen.

Auch bei Tieren treten Vergiftungserscheinungen bei mehr als 5 bis 10 µg Selen/g in der Tiernahrung auf, beispielsweise Hemmung des Wachstums, Haarausfall, Erweichung der Hörner und Hufe, bei Vögel Federausfall. Jedoch ist auch bei Tieren Selen zur Aufzucht von Küken, Puten und Schweinen notwendig, ebenso wie zur Vermeidung spezifischer Erkrankungen bei Nutztieren, insbesondere Schafen. Daher ist Natriumselenit und Natriumselenat als Mischfutterzusatz oder zur Düngung von weiden erforderlich, da der natürlicher Selengehalt tierischer und pflanzlicher Futtermittel oft unzureichend ist oder das Element nicht ausreichend freigesetzt wird.

In der US 4 668 515 werden geschmacksverstärkte Getränke, enthaltend Na-Selenit, Ascorbinsäure und Zitronensäure offenbart; die GB 2 323 030 A betrifft Nahrungsmittel für immungeschwächte Patienten, die essentielle Aminosäuren, Vitamine und eine Selen-Quelle enthalten.

Aufgabe der vorliegenden Erfindung war es, selenhältige Präparate zu verbessern und einer Lebensmittel-/Futtermittel-technologischen sowie pharmazeutischen Verwendung zuzuführen bzw. hinsichtlich ihrer Wirkung in diesen Gebieten zu verbessern.

Diese Aufgabe wird erfindungsgemäß gelöst durch die Verwendung einer selenithältigen wässerigen Lösung, versetzt mit pharmazeutisch akzeptablen Säuren, ausgewählt aus Zitronensäure, Essigsäure, Apfelsäure, Kohlensäure, Fruchtsäuren und Mischungen davon zur Herstellung eines Medikaments zur Verhinderung oder Behandlung von viralen Erkrankungen, vorzugsweise von Herpes-Infektionen, insbesondere Herpes simplex-Infektionen.

Es hat sich gezeigt, dass die Erniedrigung des pH-Wertes von selenhältigen Lösungen, insbesondere Natriumselenit-/-selenatlösungen, ein gegenüber den nicht pH-reduzierten Lösungen stark gesteigertes antioxidatives Potential aufweisen. Die erfindungsgemäß hergestellten medikamente zeigen auch überraschende therapeutische Effekte, vor allem bei viralen Erkrankungen.

Bevorzugterweise wird daher eine wässerige Selenit- Lösung verwendet.

Als Säuerungsmittel für die selenithältige Lösung kommen säuerungsmittel in Frage, die für Mensch oder Tier unbedenklich sind, wie z.B. Zitronensäure, Essigsäure, Apfelsäure, Kohlensäure, diverse Fruchtsäuren und Mischungen davon.

Die vorliegende Erfindung betrifft auch ein Präparat als gel oder Emulsion umfassend
- eine pharmazeutisch verabreichbare oder lebensmittelkompatible Form von Selenit und
- eine pharmazeutisch akzeptable oder lebensmittelkompatible Säure, ausgewählt aus Zitronensäure, Essigsäure, Apfelsäure, Kohlensäure, diversen Fruchtsäuren und Mischungen davon.

Das erfindungsgemäße Präparat weist ein gesteigertes antioxidatives Potential auf, so dass es in der lebensmitteltechnischen/futtermitteltechnischen Anwendung positive Eigenschaften für Mensch oder Tier aufweist. Im Rahmen der pharmazeutischen Verwendung von Selenverbindungen wird durch das erfindungsgemäße Präparat auch die pharmazeutische Wirkung verbessert oder gar neue pharmazeutische Verwendungen für Selenverbindungen erschlossen.

Bevorzugterweise wird durch die Ansäuerung des selenhältigen Mittels ein pH von unter 7,0, vorzugsweise unter 5,0, insbesondere unter 4,0, vorgesehen. Besonders bevorzugte erfindungsgemäße Mittel weisen einen pH von 6,0 bis 2,0, insbesondere von 3,0 bis 2,5, auf.

Selen wird im Rahmen des erfindungsgemäßen Präparates in Selenit-Form zur Verfügung gestellt.

Das erfindungsgemäße Präparat kann nicht nur in wässriger Lösung vorgesehen werden. Bevorzugte weitere Formen stellen die Gelform oder die Emulsion vor, die sich insbesondere für die pharmazeutische Anwendung als hervorragend geeignet herausgestellt haben, da damit eine lokale topische Anwendung ermöglicht wird.

Selbstverständlich kann das erfindungsgemäße Präparat weiters Hilfsstoffe wie Puffersubstanzen, Farbstoffe, Stabilisierungsmittel oder Trägersubstanzen und/oder weitere wirksame Komponenten, wie z.B.Antibiotika, antivirale Mittel, Antimykotika, schmerzhemmende Mittel oder entzündungshemmende Mittel enthalten, wobei diese Hilfsstoffe auch in allen möglichen Kombinationen verwendet werden können. Die jeweilige Art des Hilfsstoffes bzw. der weiteren wirksamen Komponente richtet sich nach der jeweiligen Verwendung im Einzelfall.

Das erfindungsgemäße Präparat eignet sich besonders gut zur pharmazeutischen Verwendung. Bevorzugt ist allerdings auch die Verwendung als Lebensmittel oder Lebensmittelzusatz bzw. als Futtermittel oder Futtermittelzusatz.

Bei der pharmazeutischen Anwendung des erfindungsgemäßen Präparates hat sich vor allem eine überraschende Wirksamkeit bei der Verhinderung oder Behandlung viraler Erkrankungen herausgestellt.

Bevorzugterweise wird daher das erfindungsgemäße Präparat zur Herstellung eines Medikaments zur Verhinderung oder Behandlung von Herpes-Infektionen, insbesondere Herpes simplex-Infektionen verwendet. Andererseits hat sich das erfindungsgemäße Präparat auch als überaus wirksam zur Verhinderung oder Behandlung von Pigmentflecken (hervorgerufen durch Lipofuszin-Ablagerungen) erwiesen.

Die Erfindung wird an Hand der nachfolgenden Beispiele, auf die sie jedoch selbstverständlich nicht eingeschränkt ist, näher erläutert.

### Beispiel 1 : Herstellung einer angesäuerten Natriumselenit-Lösung:

Eine angesäuerte Natriumselenit-Lösung wurde mit folgender Zusammensetzung hergestellt (pro 100 ml):

| | |
|---|---|
| Natriumselenit-Pentahydrat | 0,111 g |
| Maltodextrin | 0,5 g |
| Zitronenaroma | 0,1 g |
| Zitronensäure | 0,5 g |
| Lebensmittelfarbe | 0,01 g |
| Kaliumsorbat | 0,1 g |
| Natrium-Benzoat | 0,05 g |
| Aqua destillata | 99,29 g |

### Beispiel 2 : Behandlung von Herpes simplex-Infektionen

Zwölf erwachsene Patienten (sieben weiblich, fünf männlich) sowie acht Kinder (vier weiblich, vier männlich) wurden bei diagnostizierter Stomatitis herpetica/aphtosa mit fünf mal fünf Tropfen täglich bukkal (Kinder erhielten eine um das Zehnfache verdünnte Lösung) sowie gleichzeitiger externer Behandlung (fünf mal täglich betroffene Stellen mit den Tropfen betupfen) üblicherweise über einen Zeitraum von sieben Tagen behandelt. Sieben von acht Kindern bekamen zusätzlich zur antioxidativen Selen-Therapie Lokalanästhetika und/oder Antibiotika und/oder Antimykotika und/oder schmerz- und entzündungshemmende Medikamente, nicht jedoch eine zusätzliche antivirale Therapie verordnet.

Neun der zwölf Erwachsenen wurden ausschließlich mit den stark antioxidativen Selen-Tropfen behandelt, einer von zwölf Patienten erhielt zusätzlich zu den Tropfen ein antivirales Medikament (Aciclovir). Die Ergebnisse sind in der nachfolgenden Tabelle dargestellt.

| **Herpes labialis-Therapie** | | | | | |
|---|---|---|---|---|---|
| **Patient Nr.** | **Geschlecht** | **Häufigkeit** | **bisherige Behandlung** | **Nebenwirkungen** | **Behandlungs erfolg** |
| 1 | m | 1j | lokale Sal- | keine | 1 |
| | | | bentherapie | | |
| 2 | m | mtl. | Aciclovir | keine | 1 |
| 3 | m | 3-5j | Aciclovir | keine | 1 |
| 4 | w | 3j | Aciclovir | keine | 1 |
| 5 | m | - | - | - | 1 |
| 6 | w | 3j | Famvir | keine | 2 |
| 7 | w | mtl. | Aciclovir | keine | 1 |
| 8 | w | 3-5j | Aciclovir | keine | 1 |
| 9 | w | 3 j | Aciclovir | keine | 1 |
| 10 | w | 3j | Aciclovir | keine | 1 |

| **Stomatis herpetica/aphtosa-Therapie** | | | | | |
|---|---|---|---|---|---|
| **Patient Nr.** | **Geschlecht** | **Häufigkeit** | **bisherige Behandlung** | **Nebenwirkungen** | **Behandlungerfolg** |
| 11 | m | - | Xylocain | keine | 1 |
| 12 | w | - | - | keine | 2 |
| 13 | w | - | - | keine | 1 |
| 14 | m | - | - | keine | 1 |
| 15 | m | - | - | keine | 1 |
| 16 | w | - | - | keine | 2 |
| 17 | m | - | - | keine | 1 |
| 18 | w | - | - | keine | 2 |
| 19 | w | - | - | keine | 2 |
| 20 | m | mtl. | - | keine | 2 |
| m = männlich | | | | | |
| w = weiblich | | | | | |
| mtl. = monatlich | | | | | |
| 1j = 1 x jährlich | | | | | |
| 2j = 2 x jährlich | | | | | |
| 3j = 3 x jährlich | | | | | |
| 3-5j = 3 bis 5 x jährlich | | | | | |
| 1 = sehr gut | | | | | |
| 2 = gut | | | | | |
| 3 = nicht ausreichend | | | | | |

Die Untersuchungsberichte des Prüfarztes zeigen bei neunzehn von zwanzig Fällen einen ausgezeichneten Therapieerfolg (Verschwinden des Juckreizes, Abheilung der Bläschen) bereits nach drei bis sieben Tagen. Jene PatientInnen, die an Herpesrezidiven litten, zeigten nach bukkaler und externer Anwendung der Tropfen eine deutliche Verbesserung der Rezidivrate (Verlängerung der rezidivfreien Intervalle) bzw. ein völliges Verschwinden von Rezidiven im Vergleich zu einer Therapie mit Aciclovir.

### Beispiel 3 : Behandlung von Pigmentflecken

Pigmentflecken (sogenannte Altersflecken) sind die Folge einer vermehrten Ablagerung radikalisch und peroxidisch zerstörter Eiweiß-, Fettsäure-, und Membran-Fett-Strukturen im subkutanen Gewebe und zeigen sich als örtlich abgegrenzte, hell- bis dunkelbraune, etwa stecknadelgroße Verfärbungen. Drei erwachsene Personen (zwei weiblich, eine männlich) wendeten die beschriebenen Selen-Tropfen (fünf mal täglich fünf bis zehn Tropfen an die betroffenen Stellen auf dem Handrücken einreiben) über einen Zeitraum von zwei Monaten an. Die Anwendung führte zu einer deutlichen Verringerung der Anzahl der Pigmentflecken bzw. zu einem Aufhellen dunkler Pigmentflecken.

## Patentansprüche

1. Verwendung einer selenithältigen wässerigen Lösung, versetzt mit pharmazeutisch akzeptablen Säuren, ausgewählt aus Zitronensäure, Essigsäure, Apfelsäure, Kohlensäure, Fruchtsäuren und Mischungen davon zur Herstellung eines Medikaments zur Verhinderung oder Behandlung von viralen Erkrankungen, vorzugsweise von Herpes-Infektionen, insbesondere Herpes simplex-Infektionen.

2. verwendung einer selenithältigen wässerigen Lösung, versetzt mit pharmazeutisch akzeptablen Säuren, ausgewählt aus Zitronensäure, Essigsäure, Apfelsäure, Kohlensäure, Fruchtsäuren und Mischungen davon zur Herstellung eines Medikaments zur Verhinderung oder Behandlung von Pigmentflecken.

3. Pharmazeutisches Präparat, umfassend eine pharmazeutisch verabreichbare Form von Selen als Selenit, **dadurch gekennzeichnet, dass** das Präparat als Gel oder Emulsion vorliegt und eine pharmazeutisch akzeptable Säure, ausgewählt aus Zitronensäure, Essigsäure, Apfelsäure, Kohlensäure, Fruchtsäuren und Mischungen davon, enthält.

4. Verwendung nach Anspruch 1 oder 2, **dadurch gekennzeichnet, dass** das Medikament in Gelform oder Emulsion vorliegt, wobei das Medikament lokal anzuwenden ist.

## Claims

1. The use of a selenite-containing aqueous solution, supplemented with pharmaceutically acceptable acids selected from citric acid, acetic acid, malic acid, carbonic acid, fruit acids and mixtures thereof for the production of a drug for the prevention or treatment of viral diseases, preferably herpes infections, in particular *herpes simplex* infections.

2. The use of a selenite-containing aqueous solution, supplemented with pharmaceutically acceptable acids selected from citric acid, acetic acid, malic acid, carbonic acid, fruit acids and mixtures thereof for the production of a drug for preventing or treating pigmental moles.

3. A pharmaceutical preparation, comprising a pharmaceutically administrable form of selenium as selenite, **characterised in that** the preparation is provided as a gel or emulsion and contains a pharmaceutically acceptable acid selected from citric acid, acetic acid, malic acid, carbonic acid, fruit acids and mixtures thereof.

4. The use according to claim 1 or 2, **characterised in that** the drug is provided in the form of a gel or emulsion, wherein said drug is to be applied locally.

## Revendications

1. Utilisation d'une solution aqueuse contenant du sélénite, mélangée avec des acides pharmaceutiquement acceptables, choisis parmi l'acide citrique, l'acide acétique, l'acide malique, l'acide carbonique, les acides de fruits et leurs mélanges pour la préparation d'un médicament pour inhiber ou traiter des maladies virales, de préférence des infections à l'herpès, en particulier des infections à Herpès simplex.

2. Utilisation d'une solution aqueuse contenant du sélénite, mélangée avec des acides pharmaceutiquement acceptables, choisis parmi l'acide citrique, l'acide acétique, l'acide malique, l'acide carbonique, les acides de fruits et leurs mélanges pour la production d'un médicament destiné à inhiber ou à traiter des tâches pigmentaires.

3. Préparation pharmaceutique comprenant une forme pharmaceutiquement administrable de sélénium sous la forme de sélénite, **caractérisée en ce que** la préparation se présente sous forme de gel ou d'émulsion, et un acide pharmaceutiquement acceptable choisi parmi l'acide citrique, l'acide acétique, l'acide malique, l'acide carbonique, les acides de fruits et leurs mélanges.

4. Utilisation selon la revendication 1 ou 2, **caractérisée en ce que** le médicament se présente sous forme de gel ou d'émulsion, le médicament étant utilisé par voie locale.
